# EUROPEAN PATENT APPLICATION

(11) **EP 4 154 820 A1**
(43) Date of publication of application: **29.03.2023**
(21) Application number: 21809530.5
(22) Date of filing: 12.05.2021
(51) Int. Cl.: A61B 7/02, A61B 7/04

(54) **STETHOSCOPE**

(30) Priority: 19.05.2020 JP 2020087501
(71) Applicant: FUJIFILM Corporation, Tokyo 106-8620 (JP)
(72) Inventor: SASAKI, Tsutomu, Ashigarakami-gun, Kanagawa 258-8538 (JP)
(74) Representative: Jeffrey, Philip Michael
(86) International application number: PCT/JP2021/018097
(87) International publication number: WO 2021/235297

(57) **Abstract**

Provided is a stethoscope including: a support base having a contact surface that comes into contact with an object to be measured, a rear surface provided on a side opposite to the contact surface, and a side surface that connects the contact surface and the rear surface to each other and has a neck portion having a diameter smaller than a diameter of each of the contact surface and the rear surface; a piezoelectric film that is supported by the support base and detects a vibration caused by a sound generated from the object to be measured; a first sound absorbing material that is provided on an outer surface of the rear surface and absorbs a vibration caused by a sound coming from an outside; and a second sound absorbing material that is provided on an outer surface of the side surface on a side of the contact surface with respect to the neck portion and absorbs the vibration caused by the sound coming from the outside.

## Description

### BACKGROUND OF THE INVENTION

### 1. Field of the Invention

The present disclosure relates to a stethoscope. In particular, the present disclosure relates to an electronic stethoscope capable of outputting a detected sound as data.

### 2. Description of the Related Art

Hitherto, for a stethoscope capable of amplifying and listening to sounds, such as a heart sound and a blood flow sound, which are generated inside a living body (hereinafter, referred to as a biological sound), the following technique is known as a technique for reducing noise.

For example, JP2012-179408A discloses that a sheet or a film made of a low-friction material is provided so as to cover a surface of a housing of a stethoscope to reduce a frictional sound generated by movement of a doctor's hand or finger.

### SUMMARY OF THE INVENTION

In recent years, in stethoscopes, there is a need for restraining generation of noise caused by a joint sound generated by the user's body, such as a finger, a wrist, and an elbow, in addition to noise caused by the frictional sound generated by the movement of a user's hand or finger as described above. The joint sound may be propagated to the stethoscope by way of the user's finger and hand touching a housing of the stethoscope and become noise. It may be difficult to sufficiently restrain noise caused by the joint sound from being generated, only by providing the low-friction material on the surface of the housing of the stethoscope, as disclosed in JP2012-179408A.

The present disclosure provides a stethoscope capable of restraining generation of noise caused by a sound coming from an outside.

According to a first aspect of the present disclosure, there is provided a stethoscope comprising: a support base having a contact surface that comes into contact with an object to be measured, a rear surface provided on a side opposite to the contact surface, and a side surface that connects the contact surface and the rear surface to each other and has a neck portion having a diameter smaller than a diameter of each of the contact surface and the rear surface; a piezoelectric film that is supported by the support base and detects a vibration caused by a sound generated from the object to be measured; a first sound absorbing material that is provided on an outer surface of the rear surface and absorbs a vibration caused by a sound coming from an outside; and a second sound absorbing material that is provided on an outer surface of the side surface on a side of the contact surface with respect to the neck portion and absorbs the vibration caused by the sound coming from the outside.

According to a second aspect of the present disclosure, in the above aspect, each of the first and second sound absorbing materials may contain at least one of a urethane resin, a silicone resin, or an acrylic resin.

According to a third aspect of the present disclosure, in the above aspect, each of the first and second sound absorbing materials may consist of a gel-like material.

According to a fourth aspect of the present disclosure, in the above aspect, each of the first and second sound absorbing materials may consist of a resin foam obtained by foam-molding a resin.

According to a fifth aspect of the present disclosure, in the fourth aspect, a cell of the resin foam may have an average diameter of 50 µm or more and 300 µm or less, and a through-hole of the resin foam may have an average diameter of 10 µm or more and 150 µm or less.

According to a sixth aspect of the present disclosure, in the above aspect, in each of the first and second sound absorbing materials, a density may be 200 kg/m³ or more and 480 kg/m³ or less, a thickness may be 1 µm or more and 6 mm or less, and a pressure required to compress the thickness by 25% may be 0.03 MPa or more and 0.14 MPa or less.

According to a seventh aspect of the present disclosure, in the above aspect, a thickness of the first sound absorbing material may be thinner than a thickness of the second sound absorbing material.

According to an eighth aspect of the present disclosure, in the above aspect, the first sound absorbing material may have a thickness of 1 µm or more and 3 mm or less.

According to a ninth aspect of the present disclosure, in the above aspect, a communication unit that is housed inside the support base on a side of the rear surface with respect to the neck portion and transmits information on the vibration detected by the piezoelectric film through wireless communication may further be provided.

According to a tenth aspect of the present disclosure, in the above aspect, a protective layer that is disposed on a surface of the piezoelectric film on a side coming into contact with the object to be measured and has an acoustic impedance between an acoustic impedance of the object to be measured and an acoustic impedance of the piezoelectric film may further be provided.

According to an eleventh aspect of the present disclosure, in the above aspect, the piezoelectric film may consist of a polymer-based piezoelectric composite material obtained by dispersing piezoelectric particles in a matrix consisting of a polymer material.

According to the above aspects, the stethoscope of the present disclosure can restrain generation of noise caused by a sound coming from an outside.

### BRIEF DESCRIPTION OF THE DRAWINGS

Fig. 1 is a perspective view of a stethoscope according to an exemplary embodiment as viewed from a side of a rear surface.
Fig. 2 is a perspective view of the stethoscope according to the exemplary embodiment as viewed from a side of a contact surface.
Fig. 3 is a plan view showing an example of the contact surface of the stethoscope according to the exemplary embodiment.
Fig. 4 is a cross-sectional view taken along line A-A of Fig. 3.
Fig. 5 is a cross-sectional view taken along line A-A of Fig. 3 in a state in which the stethoscope is pressed against a living body.
Fig. 6 is a schematic view showing a configuration of a piezoelectric film and a protective layer.
Fig. 7 is a diagram illustrating a structure of a sound absorbing material.
Fig. 8 is a block diagram showing a configuration of the stethoscope according to the exemplary embodiment.
Fig. 9 is a block diagram showing a configuration of an output unit according to the exemplary embodiment.
Fig. 10 is a perspective view of a stethoscope according to a modification example as viewed from a side of a rear surface.
Fig. 11 is a plan view showing an example of a contact surface of the stethoscope according to the modification example.

### DESCRIPTION OF THE PREFERRED EMBODIMENTS

Hereinafter, examples of embodiments of the technique of the present disclosure will be described in detail with reference to the drawings. Hereinafter, description will be made by using a living body 12 as an example of an object to be measured and a biological sound as an example of a sound generated from the object to be measured. Examples of the biological sound include heartbeat, respiratory sounds, blood flow sounds, and intestinal sounds.

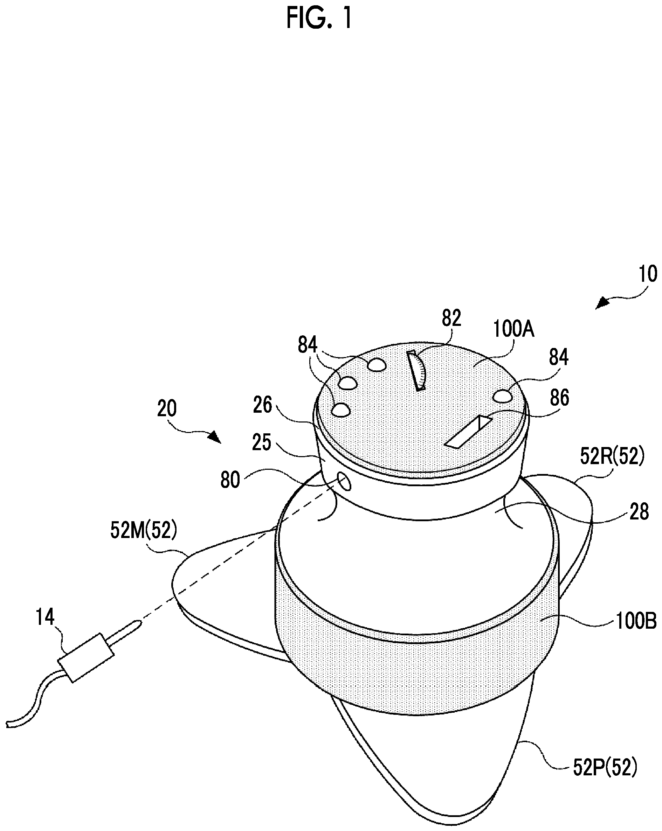

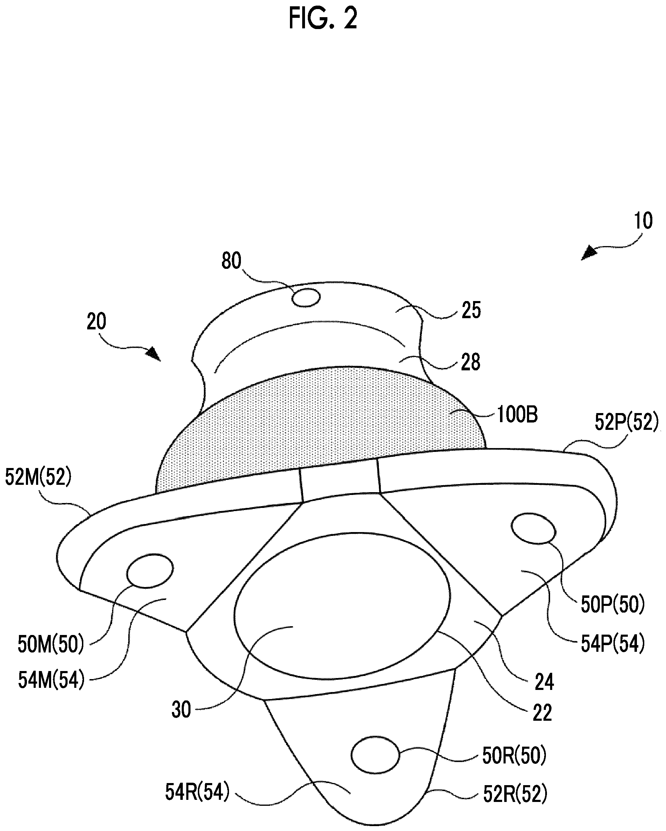

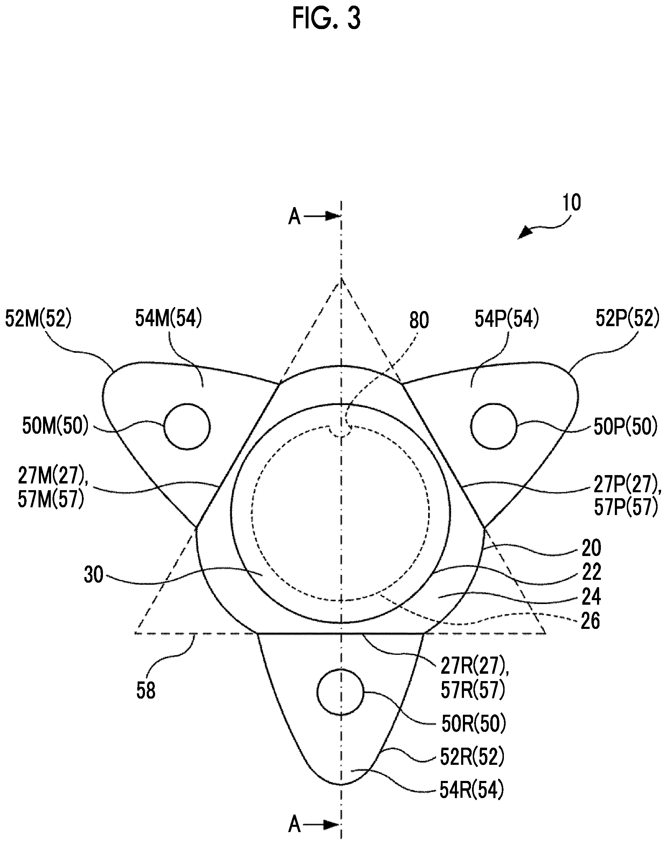

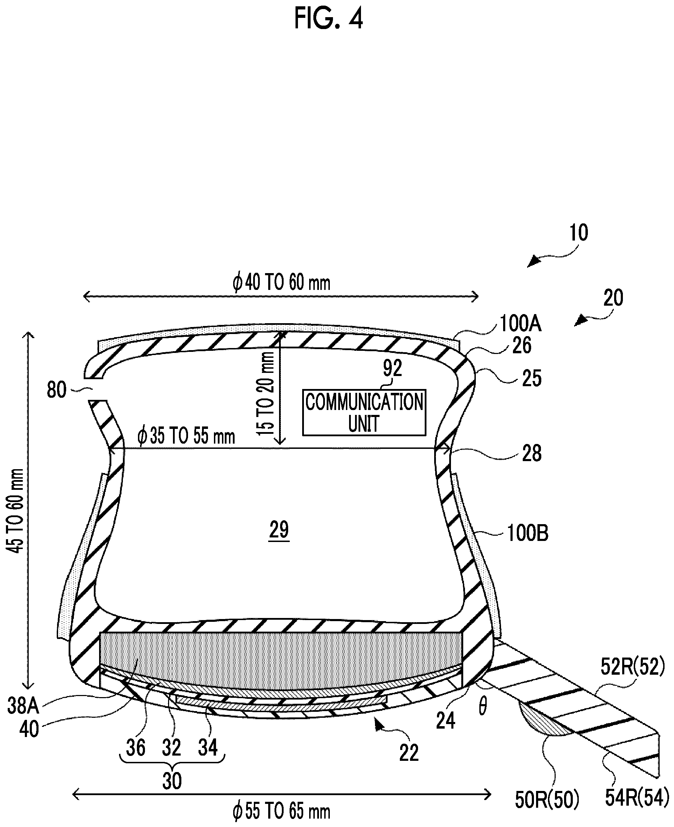

First, a configuration of a stethoscope 10 according to the present exemplary embodiment will be described with reference to Figs. 1 to 4. Fig. 1 is a perspective view of the stethoscope 10 according to the present exemplary embodiment as viewed from a side of a rear surface 26. Fig. 2 is a perspective view of the stethoscope 10 according to the present exemplary embodiment as viewed from a side of a contact surface. Fig. 3 is a plan view showing an example of the contact surface of the stethoscope 10 according to the present exemplary embodiment. Fig. 4 is a cross-sectional view taken along line A-A of Fig. 3.

As shown in Figs. 1 to 4, the stethoscope 10 comprises a support base 20, a piezoelectric film 30, a first sound absorbing material 100A, and a second sound absorbing material 100B. In addition, the stethoscope 10 comprises elastic members 52P, 52M, and 52R in which a positive electrode 50P, a negative electrode 50M, and a reference electrode 50R as an example of an electrocardiographic electrode are disposed, respectively.

The support base 20 has an opening portion 22, a first contact surface 24 that extends around the opening portion 22 and that comes into contact with the living body 12, the rear surface 26 provided on a side opposite to the first contact surface 24, and a side surface 25 that connects the first contact surface 24 and the rear surface 26 to each other. Further, the support base 20 has a neck portion 28 having a diameter smaller than a diameter of each of the first contact surface 24 and the rear surface 26 and provided on the side surface 25.

In the support base 20, the diameter of the rear surface 26 is equal to or less than the diameter of the first contact surface 24. Further, the neck portion 28 is located closer to the side of the rear surface 26 on the side surface 25. For example, as shown in Fig. 4 as an example, the diameter of the first contact surface 24 is 55 to 65 mm, the diameter of the rear surface 26 is 40 to 60 mm, and the diameter of the neck portion 28 is 35 to 55 mm. The distance between the first contact surface 24 and the rear surface 26 is 45 to 60 mm, and the distance between the rear surface 26 and the neck portion 28 is 15 to 20 mm.

The first sound absorbing material 100A is provided on the outer surface of the rear surface 26 of the support base 20, and the second sound absorbing material 100B is provided on the outer surface of the side surface 25 of the support base 20 on the side of the first contact surface 24 with respect to the neck portion 28. Further, the support base 20 is made of a member harder than the elastic members 52P, 52M, and 52R.

The support base 20 has an output terminal 80 provided on the side surface 25. The output terminal 80 is a terminal from which a signal indicating a biological sound is output (an adjustment signal S3, which will be described later). The output terminal 80 includes, for example, an earphone jack to which the terminal of an earphone 14 that is used to listen to the biological sound acquired by the stethoscope 10 is connected. The output terminal 80 is preferably disposed on the side surface 25 on the side of the rear surface 26 with respect to the neck portion 28. With such a configuration, the terminal of the earphone 14 connected to the output terminal 80 and the user's hand do not interfere with each other in a case where the user holds the side surface 25 on the side of the first contact surface 24 with respect to the neck portion 28 with the hand, so that the operability can be improved.

The support base 20 has an input unit 82, a display unit 84, and a charging terminal 86 that is used to charge a battery 96, which will be described later, on the rear surface 26. The input unit 82 is a portion in which an operation for adjusting a volume level of the biological sound heard by using the earphone 14 is performed. The input unit 82 includes, for example, a dial-type input component. The dial-type input unit 82 is employed, so that the user can be restrained from erroneously operating the input unit 82 as compared with the button-type. The display unit 84 includes, for example, an LED light and displays a remaining capacity of the battery 96 and the like.

The elastic members 52P, 52M, and 52R have second contact surfaces 54P, 54M, and 54R that come into contact with the living body 12, respectively, and are elastic members connected to the periphery of the support base 20. Hereinafter, in a case where the elastic members 52P, 52M, and 52R are not distinguished from each other, the elastic members 52P, 52M, and 52R will be referred to as an elastic member 52. In a case where the second contact surfaces 54P, 54M, and 54R are not distinguished from each other, the second contact surfaces 54P, 54M, and 54R will be referred to as a second contact surface 54.

The elastic member 52 may contain, for example, any one of an elastomer material, a silicone resin, a silicone rubber, a urethane rubber, a natural rubber, a styrene-butadiene rubber, a chloroprene rubber, an acrylonitrile rubber, a butyl rubber, an ethylene propylene rubber, a fluoro-rubber, or a chlorosulfonated polyethylene rubber. In the elastic member 52 containing these materials, the greater the thickness is and the higher the Young's modulus is, the higher the durability is and the lower the flexibility is. Low flexibility may cause discomfort due to the hardness in a case where the elastic member 52 is pressed against the living body 12.

Therefore, the elastic member 52 preferably has a thickness of 0.5 mm or more and 50 mm or less. The thickness is more preferably 3 mm or more and 30 mm or less, and most preferably 5 mm or more and 20 mm or less. Further, the elastic member 52 preferably has a Young's modulus of 0.2 MPa or more and 50 MPa or less. The Young's modulus is more preferably 0.2 MPa or more and 10 MPa or less. The elastic member 52 has the thickness and the Young's modulus within the above-described ranges, so that a durable and flexible member appropriate for use as a stethoscope can be obtained.

The positive electrode 50P, the negative electrode 50M, and the reference electrode 50R that is used to measure a reference level are disposed on the second contact surfaces 54P, 54M, and 54R, respectively. That is, each of three electrocardiographic electrodes of the positive electrode 50P, the negative electrode 50M, and the reference electrode 50R is disposed around the piezoelectric film 30. Hereinafter, in a case where the positive electrode 50P, the negative electrode 50M, and the reference electrode 50R are not particularly distinguished from each other, the positive electrode 50P, the negative electrode 50M, and the reference electrode 50R will be referred to as an electrocardiographic electrode 50.

The electrocardiographic electrode 50 is preferably attachable to and detachable from the elastic member 52. For example, the electrocardiographic electrode 50 and the elastic member 52 may be provided with a pair of coupling members (for example, a snap coupling member). Further, for example, a surface of at least one of the electrocardiographic electrode 50 or the elastic member 52, which comes into contact with each other, may be provided with an attachable and detachable adhesive member. The electrocardiographic electrode 50 is attachable and detachable, so that the electrocardiographic electrode 50 can be appropriately replaced.

As the electrocardiographic electrode 50, a commercially available disposable type electrocardiographic electrode can be used. The surface of the electrocardiographic electrode 50, which comes into contact with the living body 12, preferably has adhesiveness. Specifically, an adhesive strength measured by the following measuring method is preferably 0.25 N/mm (3 N / 12 mm) or less.

### [180° Peeling Test]

One end of a test piece having a width of 12 mm was stuck to an end of a test plate, and immediately, while pressure was applied at a speed of 1 mm/s by using a pressing roller, the test piece was stuck to the test plate such that the other end side of the test piece remains as a pulling margin. The test plate was set in a testing machine (AGS-X (manufactured by Shimadzu Corporation) or ZTA (manufactured by IMADA Co., Ltd.)), the test piece was peeled off from the test plate at a speed of 10 mm/s, and an average value of measured values (N) from after the measured value was stabilized to the end of peeling off was calculated, thereby obtaining a value of the adhesive strength.

For the cardiac electrical activity that is detected by the electrocardiographic electrode 50 and the biological sound that is detected by the piezoelectric film 30 (details will be described later), the stethoscope 10 is brought into contact with the living body 12 without moving for a certain period of time, to detect the cardiac electrical activity and the biological sound. Therefore, the electrocardiographic electrode 50 has adhesiveness on the surface that comes into contact with the living body 12, so that the stethoscope 10 can be fixed to the living body 12, and the stethoscope 10 can be restrained from moving easily. That is, the electrocardiographic electrode 50 has adhesiveness, so that the detection efficiency of the cardiac electrical activity and the biological sound can be restrained from decreasing in a case where the stethoscope 10 is brought into contact with the living body 12.

In order for the electrocardiographic electrode 50 to detect the cardiac electrical activity of the living body 12 with sufficient accuracy, it is preferable that the electrocardiographic electrode 50 has a certain area or more. Therefore, it is preferable that the second contact surface 54 of the elastic member 52 on which the electrocardiographic electrode 50 is disposed has an area of 100 mm² or more such that the electrocardiographic electrode 50 having a certain area or more can be disposed. The second contact surface 54 has an area of 100 mm² or more, so that the electrocardiographic electrode 50 having a sufficient area can be disposed, and the cardiac electrical activity can be detected with sufficient accuracy.

The electrocardiographic electrode 50 may be embedded in the elastic member 52. That is, the electrocardiographic electrode 50 may not be attachable to and detachable from the elastic member 52. In this case, for example, a member having excellent abrasion resistance, such as stainless steel, is preferably used as the electrocardiographic electrode 50.

Here, a position of the output terminal 80 according to the present exemplary embodiment will be described. In a case where the electrocardiographic electrode 50 is brought into contact with the living body 12, a direction of an electrocardiographic signal S5 (details will be described later) that is output varies depending on a positional relationship between the heart of the living body 12, and the positive electrode 50P, the negative electrode 50M, and the reference electrode 50R. In order to output the electrocardiographic signal S5 in a defined direction, it is assumed that the direction of the electrocardiographic electrode 50 with respect to the living body 12 is required to be determined such that, for example, the reference electrode 50R faces the side of the head of the living body 12 and the positive electrode 50P and the negative electrode 50M face the side of the legs of the living body 12. Therefore, it is desired that the user can easily determine which functions three electrocardiographic electrodes 50 have.

As shown in Fig. 3, the output terminal 80 is disposed on an axis A-A passing through the center of one predetermined electrocardiographic electrode 50, out of three electrocardiographic electrodes 50, and the center of the piezoelectric film 30. In the present exemplary embodiment, the electrocardiographic electrode 50 disposed on the axis A-A is the reference electrode 50R. Further, the positive electrode 50P and the negative electrode 50M are disposed around the piezoelectric film 30 at positions symmetrical to each other with respect to the axis A-A.

With such a configuration, the user can easily determine which electrocardiographic electrode 50 is the reference electrode 50R on the basis of the position of the output terminal 80. Therefore, the operability of the user can be improved.

As shown in Fig. 3, the disposition of the output terminal 80 is not limited to the position facing the reference electrode 50R with the piezoelectric film 30 interposed therebetween. For example, the output terminal 80 may be disposed on the axis A-A at a position closest to the reference electrode 50R.

Further, the electrocardiographic electrode 50 disposed on the axis A-A is not limited to the reference electrode 50R. Three electrocardiographic electrodes 50 need only be distinguished from each other on the basis of the positional relationship with the output terminal 80, and the positive electrode 50P and the negative electrode 50M may be used as the electrocardiographic electrode 50 disposed on the axis A-A.

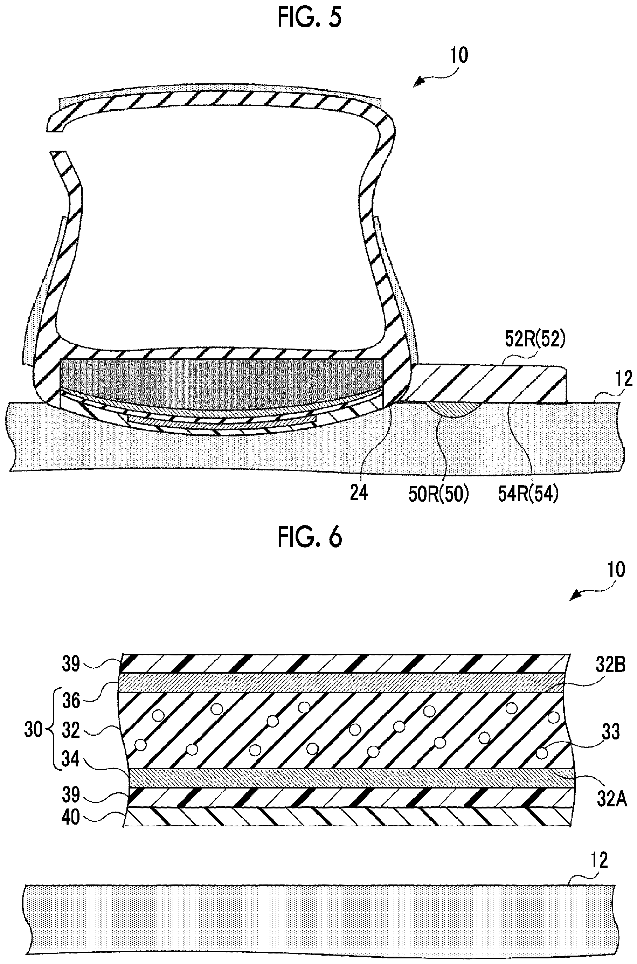

Next, the support base 20 and the elastic member 52 according to the present exemplary embodiment will be described with reference to Figs. 3 to 5. Fig. 5 shows a cross-sectional view of the stethoscope 10 shown in Fig. 3 taken along line A-A in a state in which the stethoscope 10 is pressed against the living body 12.

In order for the electrocardiographic electrode 50 to detect the cardiac electrical activity of the living body 12 with sufficient accuracy, as described above, it is necessary that the electrocardiographic electrode 50 has a certain area or more. In addition, three electrocardiographic electrodes 50 are disposed so as to be sufficiently separated from each other, so that a myoelectric influence of the living body 12 can be eliminated, and the cardiac electrical activity can be detected with a high SN ratio.

As shown in Fig. 3, the support base 20 has three first connection sides 27P, 27M, and 27R that are connected to the elastic members 52P, 52M, and 52R, respectively, as sides defining the first contact surface 24. The first connection sides 27P, 27M, and 27R form at least a part of respective sides of a triangle 58 encompassing the first contact surface 24. Hereinafter, in a case where the first connection sides 27P, 27M, and 27R are not particularly distinguished from each other, the first connection sides 27P, 27M, and 27R will be referred to as a first connection side 27.

The elastic member 52P has a second connection side 57P that is connected to the first connection side 27P of the support base 20, as a side defining the second contact surface 54P. The length of the second connection side 57P is equal to the length of the first connection side 27P. Similarly, the elastic member 52M has a second connection side 57M that is connected to the first connection side 27M of the support base 20, as a side defining the second contact surface 54M. The length of the second connection side 57M is equal to the length of the first connection side 27M. Similarly, the elastic member 52R has a second connection side 57R that is connected to the first connection side 27R of the support base 20, as a side defining the second contact surface 54R. The length of the second connection side 57R is equal to the length of the first connection side 27R.

Hereinafter, in a case where the second connection sides 57P, 57M, and 57R are not particularly distinguished from each other, the second connection sides 57P, 57M, and 57R will be referred to as a second connection side 57. Here, the length of the second connection side 57 "equal" to the length of the first connection side 27 is not limited to a case of being completely equal thereto, and the second connection side 57 need only have a length enough to be stably connected to the first connection side 27, and for example, may have a difference of about ±10%. Further, the elastic member 52 has a shape that is tapered in a direction away from the second connection side 57.

It is possible to dispose three electrocardiographic electrodes 50 so as to be sufficiently separated from each other by connecting the elastic member 52P to the support base 20 with the above-described configuration, so that a myoelectric influence of the living body 12 can be eliminated, and the cardiac electrical activity can be detected with a high SN ratio.

The triangle 58 is not limited to an equilateral triangle as shown in Fig. 3. The triangle 58 may be, for example, an isosceles triangle in which the reference electrode 50R is disposed on the base and the positive electrode 50P and the negative electrode 50M are disposed on the equal sides, respectively.

As shown in Fig. 4, an angle θ of the elastic member 52R formed by the second contact surface 54R and the first contact surface 24 is an obtuse angle in a state in which the first contact surface 24 is separated from the living body 12. As shown in Fig. 5, a pressing force with which the first contact surface 24 of the support base 20 is pressed against the living body 12 is applied to the stethoscope 10 so that the first contact surface 24 and the second contact surface 54R of the elastic member 52R extend to the same in-plane. The same applies to the elastic members 52P and 52M.

As described above, the elastic member 52 has a Young's modulus of 0.2 MPa or more and 50 MPa or less, and the support base 20 is formed of a member harder than the elastic member 52. Therefore, the elastic member 52 can bend such that the first contact surface 24 and the second contact surface 54 extend to the same in-plane, with the first connection side 27 and the second connection side 57 as a starting point. In this case, since a bias force acts in a direction in which the second contact surface 54 is pressed against the living body 12, the electrocardiographic electrode 50 and the living body 12 can be brought into close contact with each other, and the cardiac electrical activity can be detected with a high SN ratio.

Next, the configuration of the piezoelectric film 30 and a protective layer 40 according to the present exemplary embodiment will be described with reference to Figs. 4 and 6. Fig. 6 is an enlarged schematic view of a part of the piezoelectric film 30 and the protective layer 40 in order to show the configuration of the piezoelectric film 30 and the protective layer 40.

As shown in Fig. 4, the piezoelectric film 30 is supported by the support base 20 in a state in which the surface exposed from the opening portion 22 is convexly curved. Further, the surface of the piezoelectric film 30, which is exposed from the opening portion 22, protrudes with respect to the first contact surface 24.

Since the surface of the piezoelectric film 30, which is exposed from the opening portion 22, is convexly curved, it is possible to increase the expansion and contraction of the piezoelectric film 30 in the in-plane direction as compared with a case where the piezoelectric film 30 is provided in a planar shape. That is, the piezoelectric film 30 is supported by the support base 20 in a state in which the surface exposed from the opening portion 22 is convexly curved, so that it is possible to increase the amplitude of the voltage detected by the piezoelectric film 30 and sound can be collected with a high SN ratio.

The piezoelectric film 30 may be directly connected to a part of the support base 20, or may be connected to the support base 20 via, for example, another member, such as an adhesive. Further, the piezoelectric film 30 is elastic and flexible to such an extent that breakage does not occur in a case where the piezoelectric film 30 is pressed against the living body 12.

A space between the piezoelectric film 30 and the support base 20 is filled with a cushioning material 38A, and the piezoelectric film 30 is supported by the cushioning material 38A so as to be convexly curved. The cushioning material 38A has moderate elasticity, and supports the piezoelectric film 30 and applies a certain mechanical bias to the entire surface of the piezoelectric film 30. With this, the vibration of the piezoelectric film 30 generated in the thickness direction can be converted into an expansion/contraction movement of the piezoelectric film 30 in the in-plane direction, and the electric charge generation efficiency can be improved. Further, the stethoscope 10 having an appropriate repulsive force can be realized by changing the filling density of the cushioning material 38A.

The material of the cushioning material 38A need only have moderate elasticity and be suitably deformed without interfering with the vibration of the piezoelectric film 30. Specifically, for example, αGEL (registered trademark) (manufactured by Taica Corporation) containing silicone as the main raw material, wool felt containing polyester fibers, such as rayon and polyethylene terephthalate (PET), a fiber material, such as glass wool, and a foaming material, such as polyurethane, are preferably used. The space between the piezoelectric film 30 and the support base 20 may be filled with any gas instead of the cushioning material 38A.

As shown in Fig. 6, the piezoelectric film 30 comprises a piezoelectric layer 32 having, as two main surfaces facing each other, a first main surface 32A, which is a main surface on the side of the living body 12, and a second main surface 32B, which is a main surface opposite to the side of the living body 12. In addition, the piezoelectric film 30 comprises a first electrode 34 provided on the first main surface 32A of the piezoelectric layer 32 and a second electrode 36 provided on the second main surface 32B of the piezoelectric layer 32. As shown in Fig. 6, the piezoelectric film 30 may be sandwiched between insulating layers 39 containing an insulating material, such as PET.

The piezoelectric layer 32 expands and contracts in the in-plane direction in response to the biological sound emitted from the living body 12, and generates a voltage between the first electrode 34 and the second electrode 36 in response to the expansion and contraction in the in-plane direction. In the present exemplary embodiment, a polymer-based piezoelectric composite material obtained by dispersing piezoelectric particles 33 in a matrix consisting of a polymer material can be used as the piezoelectric layer 32. The piezoelectric particles 33 may be uniformly dispersed with regularity, or may be irregularly dispersed, in the matrix.

As the matrix used in the piezoelectric layer 32, for example, a polymer material having viscoelasticity at a room temperature, such as cyanoethylated polyvinyl alcohol (cyanoethylated PVA), polyvinyl acetate, polyvinylidene chloride core acrylonitrile, a polystyrene-vinyl polyisoprene block copolymer, polyvinyl methyl ketone, and polybutyl methacrylate is preferably applied.

The piezoelectric particle 33 is a particle of a piezoelectric material, and is preferably a ceramic particle having a perovskite-type crystal structure. Examples of the piezoelectric particle 33 include lead zirconate titanate, lead lanthanum zirconate titanate, barium titanate, and a solid solution of barium titanate and bismuth ferrite.

The piezoelectric layer 32 having such a configuration causes dielectric polarization in the thickness direction of the piezoelectric layer 32. In the piezoelectric layer 32 that causes dielectric polarization, it is preferable that the piezoelectric layer 32 is disposed such that a positive electric charge is generated on the side of the second main surface 32B and a negative electric charge is generated on the side of the first main surface 32A. It is known that the living body 12 is usually positively charged in many cases. Therefore, the piezoelectric layer 32 is disposed such that the negative electric charge is generated on the side of the first main surface 32A, which is a surface on the side coming into contact with the living body 12, so that the biological sound can be efficiently detected.

As the piezoelectric layer 32, for example, an organic piezoelectric film, such as polyvinylidene fluoride (PVDF), vinylidene-ethylene trifluoride copolymer (P(VDF-TrFE)), or polylactic acid, may be used. Alternatively, as the piezoelectric layer 32, an organic material, such as a polymer electret material containing, as the main component, a polymer disclosed in JP2018-191394A, JP2014-233688A, and JP2017-12270A, may be used. Examples of the organic material include polyimide, polytetrafluoroethylene, polypropylene, Teflon (registered trademark), such as polytetrafluoroethylene (PTFE) (tetrafluoride), a tetrafluoroethylene-perfluoroalkyl vinyl ether copolymer (PFA), a tetrafluoroethylene-hexafluoropropylene copolymer (FEP) (tetra-hexafluoride), and an amorphous fluoropolymer (AF), polyethylene, and cycloolefin polymers (COCs).

However, in a case where PVDF is used as the piezoelectric layer 32 instead of the polymer-based piezoelectric composite material, dielectric polarization occurs in the in-plane direction. In this case, the SN ratio may decrease as compared with the polymer-based piezoelectric composite material in which dielectric polarization occurs in the thickness direction.

The first electrode 34 and the second electrode 36 detect the expansion and contraction of the piezoelectric layer 32 generated in the in-plane direction, as the voltage. The thickness of each of the first electrode 34 and the second electrode 36 is not particularly limited, but is preferably thin in order to ensure the flexibility of the piezoelectric film 30, and is preferably 1 µm or less, for example. The thicknesses of the first electrode 34 and the second electrode 36 may be the same as or different from each other.

It is preferable that the materials of the first electrode 34 and the second electrode 36 are each a thin film of copper (Cu) or aluminum (Al) formed by vacuum evaporation in order to ensure the flexibility of the piezoelectric film 30, a conductive polymer, and the like.

Various conductors may be used as the materials of the first electrode 34 and the second electrode 36. For example, C, Pd, Fe, Sn, Ni, Pt, Au, Ag, Cr, and Mo, and an alloy thereof may be used. Alternatively, a transparent conductive film, such as indium tin oxide (ITO), indium zinc oxide (IZO), tin oxide, and zinc oxide, may be used. Alternatively, an organic conductor, such as a conductive polymer, may be used. A method for forming the electrode is also not particularly limited, and various known methods, for example, film formation through a vapor deposition method (vacuum film formation method), such as vacuum evaporation and sputtering, screen printing, and a method of pasting a foil formed of the above-described material may be used.

The size of at least one of the first electrode 34 or the second electrode 36 may be smaller than a size of the piezoelectric layer 32. In particular, as shown in Fig. 4, the first electrode 34 is preferably provided only in the central part of the first main surface 32A. The first electrode 34 may take in electromagnetic noise from the outside by functioning as an antenna, in addition to the function of detecting the biological sound emitted from the living body 12. In order to restrain the first electrode 34 from taking in electromagnetic noise, it is preferable to reduce the size of the first electrode 34 within a range in which electromagnetic noise is not easily taken in and the biological sound can be sufficiently detected. The first electrode 34 is provided only in the central part of the first main surface 32A that comes into contact with the living body 12, so that the first electrode 34 can be restrained from taking in electromagnetic noise and sound can be collected with a high SN ratio.

Since the surface of the piezoelectric film 30, which is exposed from the opening portion 22, is convexly curved and protrudes with respect to the first contact surface 24, the piezoelectric film 30 may be easily damaged in a case where the piezoelectric film 30 comes into direct contact with the living body 12, as compared with a case where the piezoelectric film 30 is provided in a planar shape. Therefore, it is preferable to dispose the protective layer 40 on the surface of the piezoelectric film 30 on the side coming into contact with the living body 12. The protective layer 40 is disposed on the surface of the piezoelectric film 30 on the side coming into contact with the living body 12, so that damage to the piezoelectric film 30 can be prevented.

Further, in a case where the piezoelectric film 30 detects the vibration of the living body 12, a large difference in acoustic impedance (unit: MRayls = kg/m²s) therebetween, which is a value intrinsic to each substance, may cause sound reflection, thereby decreasing the detection efficiency of the biological sound. That is, the ratio of noise increases and the SN ratio decreases. Therefore, in order to alleviate the difference in acoustic impedance between the living body 12 and the piezoelectric film 30, the protective layer 40 preferably has an acoustic impedance between the acoustic impedance of the living body 12 and the acoustic impedance of the piezoelectric film 30.

The acoustic impedance of the living body 12 is known to be 1.3 to 1.5 MRayls, and the acoustic impedance of the piezoelectric film 30 is 5.0 to 10.0 MRayls. Therefore, the protective layer 40 has an acoustic impedance of 1.3 MRayls or more and 5.0 MRayls or less between the acoustic impedance of the living body 12 and the acoustic impedance of the piezoelectric film 30, thereby making it advantageous to increase the SN ratio of a sound signal S2 output from the stethoscope 10. That is, the protective layer 40 with the acoustic impedance matching an acoustic impedance between the living body 12 and the piezoelectric film 30 is provided, so that sound can be restrained from being reflected and sound can be collected with a high SN ratio.

It is preferable that any one of an elastomer material, a silicone resin, a silicone rubber, a urethane rubber, a natural rubber, a styrene-butadiene rubber, a chloroprene rubber, an acrylonitrile rubber, a butyl rubber, an ethylene propylene rubber, a fluoro-rubber, or a chlorosulfonated polyethylene rubber is used as the protective layer 40. It is desired that the material of the protective layer 40 is a material that does not cause discomfort due to coldness or hardness to the living body 12 because the protective layer 40 is directly pressed against the living body 12. The above-described material is used, so that it is possible to restrain discomfort from occurring even in a case where the protective layer 40 is pressed against the living body 12.

It is desired that the protective layer 40 has abrasion resistance because the protective layer 40 is directly pressed against the living body 12. Therefore, it is preferable that the protective layer 40 has a measured value of 50 or more and 100 or less in a hardness test using a type A durometer conforming to ASTM D2240. A hardness measured by a durometer of another standard or another measurement method may be used to obtain the same hardness as the hardness in the above-described test method. The protective layer 40 has the hardness in the above-described range, so that the protective layer 40 can have appropriate abrasion resistance for use as a stethoscope.

The protective layer 40 having high stiffness may restrict the expansion and contraction of the piezoelectric layer 32, thereby making the vibration of the piezoelectric film 30 small. Therefore, the thickness of the protective layer 40 having the above-described material and hardness need only be appropriately set according to the performance, the handleability, the mechanical strength, and the like which are required for the piezoelectric film 30. Specifically, the thickness need only be about 500 µm.

The surface of the protective layer 40, which comes into contact with the living body 12, is preferably roughened. It is desirable that the protective layer 40 is easily peeled off from the skin of the living body 12 because the protective layer 40 is directly pressed against the living body 12. The roughening treatment method is not particularly limited, and various known methods, such as mechanical roughening treatment, electrochemical roughening treatment, and chemical roughening treatment, may be used. As a roughness for roughening the surface of the protective layer 40, which comes into contact with the living body 12, an arithmetic average roughness Ra is preferably 0.1 µm or more and 100 µm or less, and more preferably 0.1 µm or more and 10 µm or less. The protective layer 40 has the roughness in the above-described range, so that the protective layer 40 can be easily peeled off from the skin of the living body 12.

The protective layer 40 may be composed of a plurality of layers that are laminated such that the acoustic impedance becomes lower toward the side coming into contact with the living body 12. In this case, the materials of the plurality of layers may be different from each other, or a material consisting of at least one of the above-described materials need only be used. Alternatively, the protective layer 40 may be a layer of which the acoustic impedance is graded such that the acoustic impedance becomes lower toward the side coming into contact with the living body 12. The protective layer 40 has such configurations, so that the difference in acoustic impedance between adjacent substances can be made smaller, and sound can be collected with a higher SN ratio.

Further, the surface of the protective layer 40, which comes into contact with the living body 12, may consist of a silicone resin, and the silicone resin may have a siloxane skeleton as the main chain skeleton and at least one of a methyl group, a vinyl methyl group, or a phenyl methyl group, as a hydrophobic side chain. The surface of the living body 12 may be wet due to sweat or the like. It is desired that at least the surface that comes into contact with the living body 12 is made of a material that is not denatured by moisture because the protective layer 40 is directly pressed against the living body 12. Therefore, for example, the protective layer 40 may be composed of a plurality of layers having a layer with matching acoustic impedance and a layer consisting of a hydrophobic material provided on the surface that comes into contact with the living body 12. The surface that comes into contact with the living body 12 has hydrophobicity, so that the protective layer 40 and the piezoelectric film 30 can be restrained from being denatured even in a case where the protective layer 40 is pressed against the wet living body 12.

Meanwhile, examples of the noise coming from the outside in a case where the stethoscope 10 is used include a joint sound generated by the user's body, such as a finger, a wrist, and an elbow, in addition to a frictional sound generated by the movement of the user's hand or finger. The joint sound is propagated to the support base 20 by way of the user's finger and hand touching the support base 20, whereby the piezoelectric film 30 may detect the sound. The first sound absorbing material 100A and the second sound absorbing material 100B are provided on the outer surface of the support base 20 according to the present exemplary embodiment, as a means for restraining the generation of noise caused by such a frictional sound and joint sound.

The configuration of the first sound absorbing material 100A and the second sound absorbing material 100B according to the present exemplary embodiment will be described with reference to Figs. 1, 4, and 7. Hereinafter, in a case where the first sound absorbing material 100A and the second sound absorbing material 100B are not distinguished from each other, the first sound absorbing material 100A and the second sound absorbing material 100B will be referred to as a sound absorbing material 100.

The sound absorbing material 100 is made of a material having a function of absorbing a vibration caused by a sound coming from the outside (hereinafter, referred to as "extraneous sound"), such as a frictional sound and a joint sound as described above. In the stethoscope 10 according to the present exemplary embodiment, the user's hand or finger touches the rear surface 26 of the support base 20, and the side surface 25 of the support base 20 on the side of the first contact surface 24 with respect to the neck portion 28. Therefore, the first sound absorbing material 100A and the second sound absorbing material 100B are provided on these outer surfaces, respectively, so that the frictional sound and the joint sound as described above can be restrained from being propagated to the support base 20 by way of the user's finger and hand touching the support base 20. That is, it is possible to restrain the generation of noise caused by the extraneous sound carried on the sound heard through the stethoscope 10.

Specifically, the sound absorbing material 100 preferably consists of a polymer material and/or a gel-like material containing the polymer material as a raw material. Part of the vibrational energy of sound, that is, air, is converted into thermal energy by the friction caused by the viscosity of the polymer material (hereinafter, referred to as "thermal conversion"). That is, the polymer material is used as the sound absorbing material 100 so that the vibration caused by the extraneous sound can cause thermal conversion and sound can be absorbed, whereby noise caused by the extraneous sound can be restrained from being generated.

For example, a urethane resin, a silicone resin, and an acrylic resin can be used as the polymer material. For example, αGEL (registered trademark), βGEL (registered trademark), and θGEL (registered trademark) (all manufactured by Taica Corporation), which contain silicone as the main raw material, can be used as the gel-like material.

The sound absorbing material 100 preferably contains at least one of the polymer materials, such as a urethane resin, a silicone resin, and an acrylic resin, and a plurality of different polymer materials may be combined. Further, the materials of the first sound absorbing material 100A and the second sound absorbing material 100B may be different from each other.

Further, the thickness of the sound absorbing material 100 is preferably 1 µm or more and 6 mm or less. Generally, the thicker the sound absorbing material 100 is, the better the sound absorbing performance is. Therefore, the thickness of the sound absorbing material 100 is set to 1 µm or more so that the sound absorbing performance can be improved, thereby making it advantageous to restrain noise caused by the extraneous sound from being generated. Meanwhile, the thickness of the sound absorbing material 100 is preferably 6 mm or less in consideration of user operability.

The thickness of the first sound absorbing material 100A is preferably thinner than the thickness of the second sound absorbing material 100B, and particularly preferably 1 µm or more and 3 mm or less. As shown in Fig. 4, in the stethoscope 10 according to the present exemplary embodiment, a communication unit 92 (details will be described later) having a wireless communication means is housed in a housing portion 29 of the support base 20 on the side of the rear surface 26 with respect to the neck portion 28. Therefore, it is preferable that the thickness of the first sound absorbing material 100A is thinner than the thickness of the second sound absorbing material 100B and is particularly 3 mm or less so as not to interfere with the communication of the communication unit 92.

Further, in the stethoscope 10 according to the present exemplary embodiment, the sound absorbing material 100 is not provided on the side of the side surface 25, on which the communication unit 92 is housed, on the side of the rear surface 26 with respect to the neck portion 28 of the support base 20. Therefore, it is possible to restrain the sound absorbing material 100 from interfering with the communication of the communication unit 92.

Further, the density of the sound absorbing material 100 is preferably 200 kg/m³ or more and 480 kg/m³ or less. The density of the sound absorbing material 100 is set to 200 kg/m³ or more so that the thermal conversion of an air vibration can be promoted, thereby making it advantageous to restrain noise caused by the extraneous sound from being generated. Further, the sound absorbing material 100 can have a sufficient mechanical strength. The density of the sound absorbing material 100 is set to 480 kg/m³ or less so that the vibration propagated by the excitation of the polymer material due to the extraneous sound can be restrained from being generated, thereby making it advantageous to restrain noise caused by the extraneous sound from being generated.

Further, the pressure required to compress the thickness of the sound absorbing material 100 by 25% (hereinafter, referred to as "25% compression load") is preferably 0.03 MPa or more and 0.14 MPa or less. The 25% compression load of the sound absorbing material 100 is set to 0.03 MPa or more so that the thermal conversion of the air vibration can be promoted, thereby making it advantageous to restrain noise caused by the extraneous sound from being generated. Further, the sound absorbing material 100 can have a sufficient mechanical strength. The 25% compression load of the sound absorbing material 100 is set to 0.14 MPa or less so that the vibration propagated by the excitation of the polymer material due to the extraneous sound can be restrained from being generated, thereby making it advantageous to restrain noise caused by the extraneous sound from being generated.

Further, the sound absorbing material 100 more preferably consists of a resin foam obtained by foam-molding a resin, and is particularly preferably a foam having an open-cell structure or a semi-open-cell structure. As the resin foam, for example, a polyurethane foam, such as PORON (registered trademark) (manufactured by Rogers Corporation), and a foam gel-like material containing silicone as the main raw material, such as NPGEL (registered trademark) (manufactured by Taica Corporation), can be used.

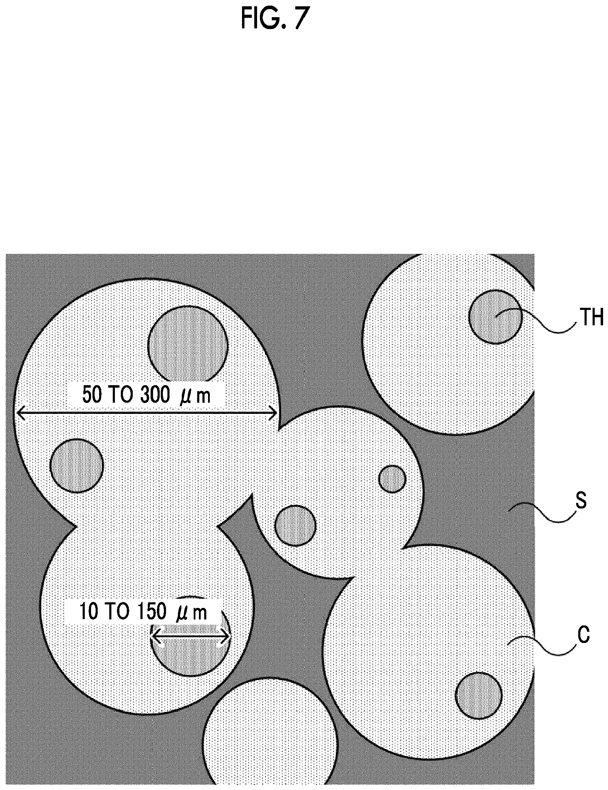

Fig. 7 is a schematic diagram showing the structure of a resin foam having a semi-open-cell structure. As shown in Fig. 7, the resin foam having a semi-open-cell structure includes a skeleton S consisting of a resin, a plurality of cells C (cells) dispersed in a resin matrix, and a through-hole TH provided between adjacent cells C.

As described above, one of sound absorption mechanisms is that part of the vibrational energy of sound, that is, air, causes thermal conversion because of the friction with a polymer material (resin). Therefore, in a case where the friction between the resin and the vibrating air propagated through the inside of the sound absorbing material 100 is promoted, the sound absorbing performance is improved. In the resin foam, since the surface area of the resin is increased by the cells C, the friction between the skeleton S, and the vibrating air propagated through the cells C and the through-hole TH is promoted, and the vibration of air easily causes thermal conversion.

In addition, in the resin foam having the open-cell structure and the semi-open-cell structure, in a case where the air vibration is propagated through the cells C and the through-hole TH having a smaller diameter than the cell C, the expansion and compression are repeated, and the air is attenuated by the viscous resistance of the air (hereinafter, referred to as "viscous attenuation"). That is, the resin foam is used as the sound absorbing material 100 so that thermal conversion and viscous attenuation of the air vibration caused by the extraneous sound can be promoted, thereby making it advantageous to absorb sound. Accordingly, noise caused by the extraneous sound can be restrained from being generated.

In a case where the resin foam is used as the sound absorbing material 100, the average diameter of the cells C is preferably 50 µm or more and 300 µm or less. The average diameter of the cells C is set to 50 µm or more so that the surface area of the resin can be sufficiently increased and the efficiency of thermal conversion can be enhanced, thereby making it advantageous to restrain noise caused by the extraneous sound from being generated. The average diameter of the cells C is set to 300 µm or less, so that the sound absorbing material 100 can have a sufficient mechanical strength. Here, the average diameter of the cells C indicates the average of the longest lengths of the cells C.

In a case where the resin foam having an open-cell structure or a semi-open-cell structure is used as the sound absorbing material 100, the average diameter of the through-holes TH is preferably 10 µm or more and 150 µm or less. The average diameter of the through-holes TH is set to 10 µm or more so that the air vibration is easily propagated to the adjacent cells C and easily causes thermal conversion, thereby making it advantageous to restrain noise caused by the extraneous sound from being generated. The average diameter of the through-holes TH is set to 150 µm or less so that the viscous attenuation of the air vibration can be promoted because the air vibration is greatly compressed in a case where the air vibration passes through the through-holes TH, thereby making it advantageous to restrain noise caused by the extraneous sound from being generated. Here, the average diameter of the through-holes TH indicates the average of the longest lengths of the through-holes TH.

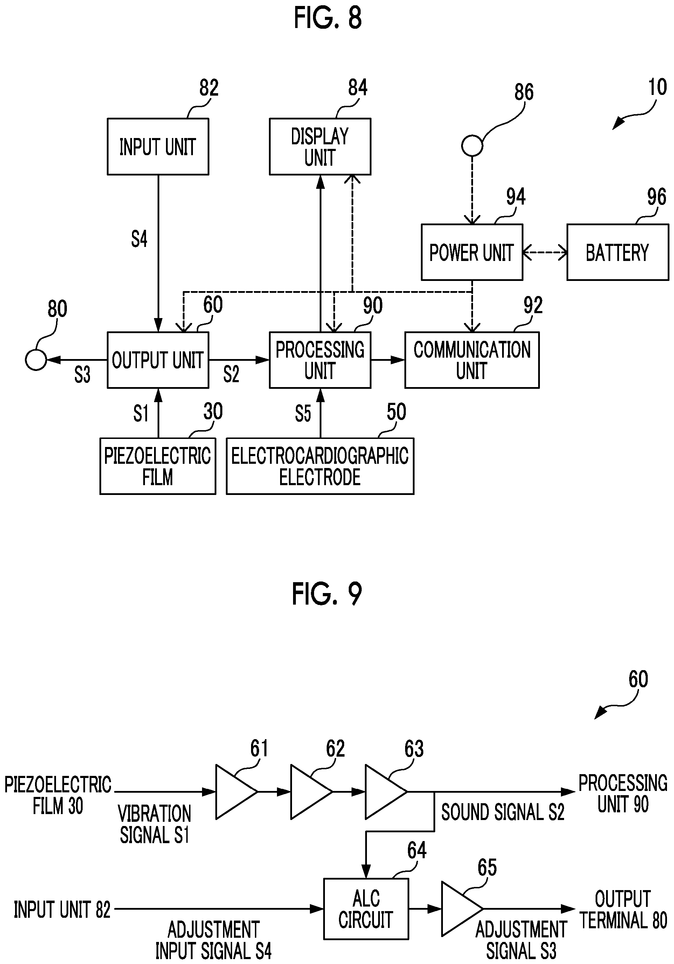

Next, the function of the stethoscope 10 according to the present exemplary embodiment will be described with reference to Fig. 8. As shown in Fig. 8, the stethoscope 10 comprises the piezoelectric film 30, the electrocardiographic electrode 50, the output terminal 80, the input unit 82, the display unit 84, and the charging terminal 86. In addition, the stethoscope 10 comprises an output unit 60, a processing unit 90, the communication unit 92, a power unit 94, and the battery 96. The output unit 60, the processing unit 90, the communication unit 92, the power unit 94, and the battery 96 may be housed in the housing portion 29 which is a space provided in the support base 20 (see Fig. 4), and may be provided outside the support base 20.

The piezoelectric film 30 detects the vibration caused by the biological sound generated from the living body 12, and outputs a vibration signal S1 to the output unit 60 on the basis of the vibration. Specifically, when the living body 12 is brought into contact with the piezoelectric film 30 in a case where a surface of the living body 12 is vibrated by the biological sound generated from the living body 12, the piezoelectric film 30 is also vibrated in response to the vibration. The piezoelectric film 30 detects the vibration as a voltage generated between the first electrode 34 and the second electrode 36, and outputs the detected voltage to the output unit 60 as the vibration signal S1.

The input unit 82 receives an adjustment input of the level of the sound signal S2 output from the output unit 60, which will be described later, and outputs an adjustment input signal S4 indicating information on the received adjustment input, to the output unit 60.

The output unit 60 outputs the sound signal S2 to the processing unit 90 on the basis of the vibration signal S1. Further, the output unit 60 adjusts the level of the sound signal S2 according to a level change of the vibration signal S1 and the adjustment input signal S4, and outputs the adjusted signal to the output terminal 80 as the adjustment signal S3. The output terminal 80 outputs the adjustment signal S3 to the outside.

The electrocardiographic electrode 50 detects the cardiac electrical activity of the living body 12. Specifically, the electrocardiographic electrode 50 is brought into contact with the vicinity of the heart of the living body 12 to detect potential on a body surface of the living body 12, and outputs the detected potential to the processing unit 90 as an electrocardiographic signal S5.

The processing unit 90 performs predetermined processing on data based on the sound signal S2 and the electrocardiographic signal S5, and outputs the processed data to the communication unit 92. The processing unit 90 may comprise an amplifier circuit, a filter circuit, and the like, and may amplify the sound signal S2 and the electrocardiographic signal S5 or extract a specific frequency. Further, the data based on the sound signal S2 and the electrocardiographic signal S5 may be output as analog data or digital data. The processing unit 90 may be composed of a microcomputer including, for example, a central processing unit (CPU), a read only memory (ROM), and a random access memory (RAM).

The communication unit 92 comprises a wireless communication means, and transmits the biological sound and electrocardiographic data to an external device. The communication means may be, for example, Bluetooth (registered trademark), Wi-Fi (registered trademark), or infrared communication, and the external device may be, for example, a personal computer or a smartphone.

The power unit 94 charges the battery 96 with electric power supplied from the charging terminal 86. Further, the power unit 94 supplies the electric power, with which the battery 96 is charged, to the output unit 60, the display unit 84, the processing unit 90, and the communication unit 92.

Further, the processing unit 90 acquires information indicating the remaining battery capacity of the battery 96 via the power unit 94, and may perform control to, for example, turn on, off, and on and off LED light of the display unit 84, on the basis of the acquired information. In this case, the user can grasp the remaining battery capacity of the battery 96 from the light emission state of the LED light. Similarly, the processing unit 90 may, for example, control the display unit 84 in a case where the sound signal S2 and the electrocardiographic signal S5 are normally acquired. In this case, the user can grasp the acquisition state of the sound signal S2 and the electrocardiographic signal S5 from the display mode of the display unit 84.

Next, the function of the output unit 60 will be described with reference to Fig. 9. As shown in Fig. 9, the output unit 60 comprises a buffer 61, a filter circuit 62, amplifiers 63 and 65, and an automatic level control (ALC) circuit 64.

The vibration signal S1 detected by the piezoelectric film 30 is input to the filter circuit 62 via the buffer 61 and noise is cut by the filter circuit 62, and then the signal is amplified by the amplifier 63 and output to the processing unit 90 and the ALC circuit 64 as the sound signal S2. The filter circuit 62 is, for example, a low-pass filter.

In a case where the stethoscope 10 is brought into contact with the living body 12 and then the stethoscope 10 is removed from the living body 12 after auscultation, the piezoelectric film 30 is greatly vibrated, and sharp noise (spike noise) may be mixed into the vibration signal S1. In a case where the spike noise is output as the sound signal S2 from the output terminal 80 as it is, the sound heard from the earphone that is connected to the output terminal 80 may become a harsh sound. In that respect, it is desirable to reduce the spike noise with the ALC circuit 64.

The adjustment input signal S4 from the input unit 82 and the sound signal S2 are input to the ALC circuit 64. The ALC circuit 64 may include, for example, a Schottky barrier diode that is used to detect spike noise from the sound signal S2, a capacitor that is used to smooth the sound signal S2, and a metal-oxide-semiconductor field-effect transistor (MOSFET) that is driven in a case where the spike noise is detected. The ALC circuit 64 uses these elements to reduce the spike noise and to adjust the level of the sound signal S2 on the basis of the adjustment input signal S4.

The sound signal S2 of which the spike noise is reduced and the level is adjusted by the ALC circuit 64 is amplified by the amplifier 65 and output to the output terminal 80 as the adjustment signal S3.

As described above, the stethoscope 10 according to the present exemplary embodiment comprises the support base 20 having the first contact surface 24 that comes into contact with the living body 12, the rear surface 26 provided on the side opposite to the first contact surface 24, and the side surface 25 that connects the first contact surface 24 and the rear surface 26 to each other and has the neck portion 28 having a diameter smaller than a diameter of each of the first contact surface 24 and the rear surface 26. In addition, the stethoscope 10 comprises the piezoelectric film 30 that is supported by the support base 20 and that detects a vibration caused by a sound generated from the living body 12. In addition, the stethoscope 10 comprises the first sound absorbing material 100A that is provided on the outer surface of the rear surface 26 and that absorbs the vibration caused by the sound coming from the outside, and the second sound absorbing material 100B that is provided on the outer surface of the side surface 25 on the side of the first contact surface 24 with respect to the neck portion 28 and that absorbs the vibration caused by the sound coming from the outside. Therefore, it is possible to restrain the generation of noise caused by the sound coming from the outside, such as a frictional sound and a joint sound.

In the stethoscope 10 according to the present exemplary embodiment, the piezoelectric film 30 is supported by the support base 20 in a state in which the surface exposed from the opening portion 22 is convexly curved, and the protective layer 40 having an acoustic impedance between the acoustic impedance of the living body 12 and the acoustic impedance of the piezoelectric film 30 is provided on the surface of the piezoelectric film 30 on the side coming into contact with the living body 12. Therefore, the biological sound can be detected with a high SN ratio.

Further, in the stethoscope 10 according to the present exemplary embodiment, the output terminal 80 is disposed on the axis passing through the center of the one predetermined electrocardiographic electrode, out of three electrocardiographic electrodes 50, and the center of the piezoelectric film 30. Therefore, the operability of the user can be improved.

Further, in the stethoscope 10 according to the present exemplary embodiment, the elastic member 52 is an elastic member that has an obtuse angle formed with the first contact surface 24, and that is connected to the first connection side 27 in the second connection side 57 having a length equal to the length of the first connection side 27 out of the sides defining the second contact surface 54. Therefore, the electrocardiographic electrode 50 can have a predetermined area while restraining the size of the stethoscope 10 itself from increasing, and three electrocardiographic electrodes 50 can be disposed so as to be sufficiently separated from each other. That is, it is possible to detect the cardiac electrical activity with a high SN ratio while improving the operability of the user.

### [Modification Example]

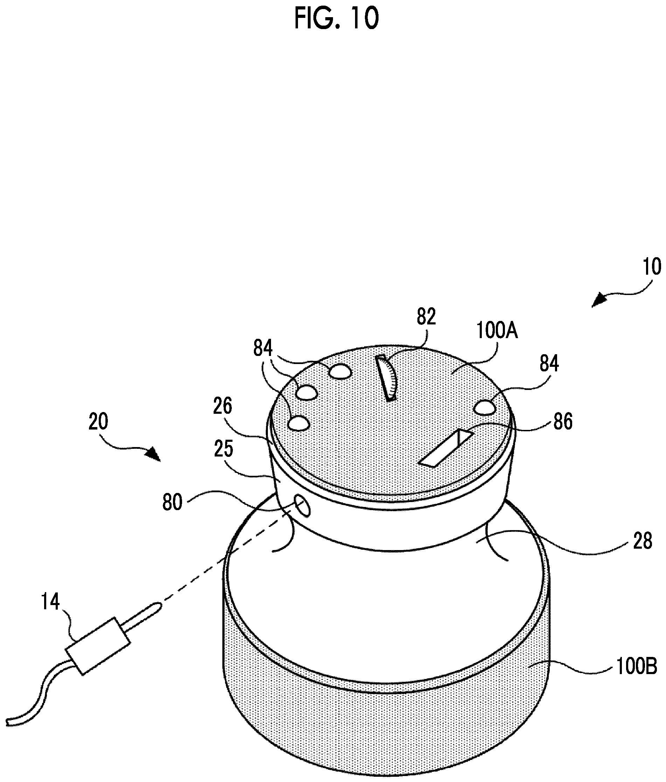

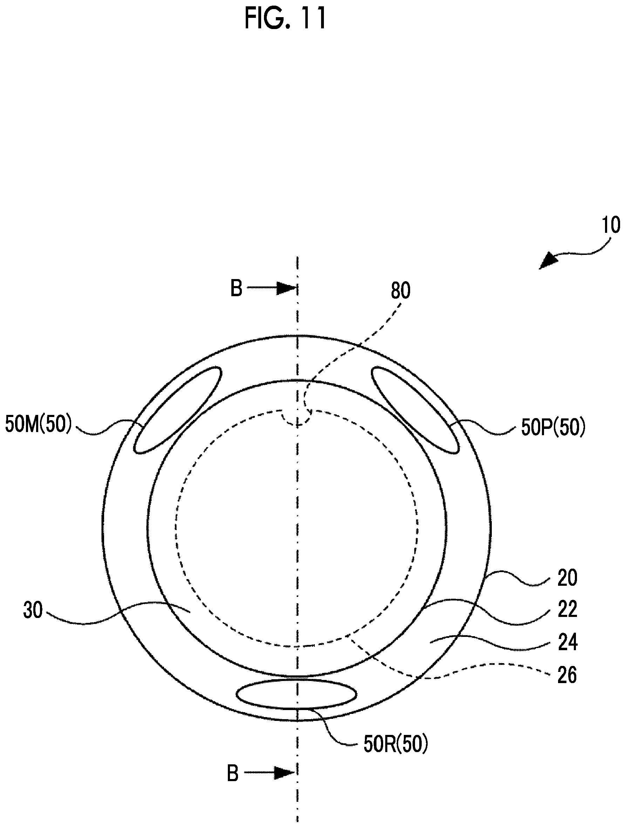

Next, the modification example of the stethoscope 10 of the present disclosure will be described with reference to Figs. 10 and 11. In the above-described exemplary embodiment, an aspect has been described in which the stethoscope 10 comprises the elastic member 52 and the electrocardiographic electrode 50 is disposed on the elastic member 52, but the configuration of the stethoscope 10 of the present disclosure is not limited thereto. Fig. 10 is a perspective view of a stethoscope 10 according to the present modification example as viewed from the side of the rear surface 26. Fig. 11 is a plan view showing an example of the contact surface of the stethoscope 10 according to the present modification example. The same reference numerals are given to the same configurations as in the above-described exemplary embodiment, and the description thereof is omitted.

As shown in Figs. 10 and 11, the stethoscope 10 in the present modification example does not comprise the elastic member 52 according to the above-described exemplary embodiment. The positive electrode 50P, the negative electrode 50M, and the reference electrode 50R are disposed on the first contact surface 24 of the support base 20, respectively. These electrocardiographic electrodes 50 may be attachably and detachably provided on the first contact surface 24, or may be embedded therein. According to the present modification example, the size of the stethoscope 10 can be reduced as compared with the stethoscope 10 according to the above-described exemplary embodiment.

As shown in Fig. 11, the output terminal 80 is disposed on an axis B-B passing through the center of one predetermined electrocardiographic electrode 50 (the reference electrode 50R in the example of Fig. 11), out of three electrocardiographic electrodes 50, and the center of the piezoelectric film 30. Therefore, as in the above-described exemplary embodiment, since the user can easily determine which electrocardiographic electrode 50 is the reference electrode 50R on the basis of the position of the output terminal 80, the operability of the user can be improved.

In the stethoscope 10 according to the exemplary embodiment and the modification example described above, the object to be measured is not limited to the living body 12, and the object to be measured may be a machine, a pipe, and the like. That is, a sound generated from the machine, the pipe, and the like is detected as the sound generated from the object to be measured so that the stethoscope can also be used for, for example, the detection of an abnormality of the machine and the pipe.

Further, in the exemplary embodiment and the modification example described above, an aspect has been described in which the stethoscope 10 comprises the electrocardiographic electrodes 50, but the present disclosure is not limited thereto. The first sound absorbing material 100A and the second sound absorbing material 100B of the present disclosure can also be applied to the stethoscope 10 in which the electrocardiographic electrode 50 is not provided.

The disclosure of Japanese patent application 2020-087501 filed on May 19, 2020 is incorporated herein by reference in its entirety. All documents, patent applications, and technical standards described in the present specification are incorporated in the present specification by reference to the same extent as in a case where the individual documents, patent applications, and technical standards were specifically and individually stated to be incorporated by reference.

## Claims

1. A stethoscope comprising:
a support base having a contact surface that comes into contact with an object to be measured, a rear surface provided on a side opposite to the contact surface, and a side surface that connects the contact surface and the rear surface to each other and has a neck portion having a diameter smaller than a diameter of each of the contact surface and the rear surface;
a piezoelectric film that is supported by the support base and detects a vibration caused by a sound generated from the object to be measured;
a first sound absorbing material that is provided on an outer surface of the rear surface and absorbs a vibration caused by a sound coming from an outside; and
a second sound absorbing material that is provided on an outer surface of the side surface on a side of the contact surface with respect to the neck portion and absorbs the vibration caused by the sound coming from the outside.

2. The stethoscope according to claim 1, wherein each of the first and second sound absorbing materials contains at least one of a urethane resin, a silicone resin, or an acrylic resin.

3. The stethoscope according to claim 1 or 2, wherein each of the first and second sound absorbing materials consists of a gel-like material.

4. The stethoscope according to any one of claims 1 to 3, wherein each of the first and second sound absorbing materials consists of a resin foam obtained by foam-molding a resin.

5. The stethoscope according to claim 4, wherein
a cell of the resin foam has an average diameter of 50 µm or more and 300 µm or less, and
a through-hole of the resin foam has an average diameter of 10 µm or more and 150 µm or less.

6. The stethoscope according to any one of claims 1 to 5, wherein in each of the first and second sound absorbing materials,
a density is 200 kg/m³ or more and 480 kg/m³ or less,
a thickness is 1 µm or more and 6 mm or less, and
a pressure required to compress the thickness by 25% is 0.03 MPa or more and 0.14 MPa or less.

7. The stethoscope according to any one of claims 1 to 6, wherein a thickness of the first sound absorbing material is thinner than a thickness of the second sound absorbing material.

8. The stethoscope according to any one of claims 1 to 7, wherein the first sound absorbing material has a thickness of 1 µm or more and 3 mm or less.

9. The stethoscope according to any one of claims 1 to 8, further comprising:
a communication unit that is housed inside the support base on a side of the rear surface with respect to the neck portion and transmits information on the vibration detected by the piezoelectric film through wireless communication.

10. The stethoscope according to any one of claims 1 to 9, further comprising:
a protective layer that is disposed on a surface of the piezoelectric film on a side coming into contact with the object to be measured and has an acoustic impedance between an acoustic impedance of the object to be measured and an acoustic impedance of the piezoelectric film.

11. The stethoscope according to any one of claims 1 to 10, wherein the piezoelectric film consists of a polymer-based piezoelectric composite material obtained by dispersing piezoelectric particles in a matrix consisting of a polymer material.
